# EUROPEAN PATENT SPECIFICATION

(11) **EP 2 326 337 B1**
(45) Date of publication and mention of the grant of the patent: **20.05.2015**
(21) Application number: 09745100.9
(22) Date of filing: 21.09.2009
(51) Int. Cl.: A61K 36/282, A61K 36/53, A61K 36/235, A61P 15/10

(54) **PREPARATION FOR THE TREATMENT OF THE ERECTILE DISORDER**
ZUBEREITUNG FÜR DIE BEHANDLUNG VON EREKTILER DYSFUNKTION
PRÉPARATION DESTINÉE AU TRAITEMENT DES TROUBLES DE L'ÉRECTION

(30) Priority: 24.09.2008 IT MO20080245
(43) Date of publication of application: 01.06.2011
(73) Proprietor: Canalini, Maria Grazia, 41024 Le Piane di Lama Mocogno (IT)
(72) Inventor: Canalini, Maria Grazia, 41024 Le Piane di Lama Mocogno (IT)
(74) Representative: Brunacci, Marco
(86) International application number: PCT/IB2009/006910
(87) International publication number: WO 2010/035101

(56) References cited:
- WO-A-89/06966
- US-A1- 2004 180 104
- THARAKAN BINU ET AL: "Botanical therapies in sexual dysfunction" PHYTOTHERAPY RESEARCH, JOHN WILEY & SONS LTD. CHICHESTER, GB, vol. 19, no. 6, 1 June 2005 (2005-06-01), pages 457-463, XP002438019 ISSN: 0951-418X
- AGLAROVA A M ET AL: "Biological characteristics and useful properties of tarragon (Artemisia dracunculus L.) (review)" PHARMACEUTICAL CHEMISTRY JOURNAL, KLUWER ACADEMIC PUBLISHERS-CONSULTANTS BUREAU, NE, vol. 42, no. 2, 10 August 2008 (2008-08-10), pages 81-86, XP019613017 ISSN: 1573-9031

## Description

### Technical Field

The present invention relates to a preparation for the treatment of the erectile disorder.

### Background Art

It is known that the erectile disorder, meaning the inability of a man to reach and/or maintain an erection adequate enough to have satisfactory sexual intercourse, is a disorder affecting millions of men in the world.

In recent years, in fact, in-depth clinical studies have shown to an increasing extent that numerous people suffer from this disorder and that the causes are numerous and different and are not necessarily the direct consequence of ageing.

The erection, in fact, is a physiological process involving different psychological, neurological, vascular and hormone aspects and the factors therefore that can affect the normal erectile function are numerous.

Furthermore, it cannot be ruled out that an important cause is to be sought in environment pollution.

Many in fact are the chemical substances foreign to the body and present in the air, in the water and in food, substances which if absorbed on a daily basis can to a considerable extent negatively affect the homeostasis of a man's body.

In particular, such substances can alter the nervous centres, the hypothalamus in particular, and the normal activity of the pituitary gland, a gland directly involved in and the main controller of male sexual activity.

Following the spreading of this disorder, therapeutic treatments have been developed of various kinds in relation to the different possible causes responsible for it.

Some therapies combat causes of the disorder of a psychological, behavioural or relational nature, and can involve a simple dialectic discussion, activities relating to the recovery of bodily sensorial perceptions, activities relating to the recovery of communication between couples or also the targeted intervention of a specialised psychologist.

Therapies of a naturopathic or nutritional rebalancing nature intervene on wrong nutritional choices of the person which are often at the bottom of the erectile disorder such as, for example, eating over-elaborate or processed foods, eating too fast and eating food that is not easily digestible, low intake of foods such as fruit and vegetables.

Other therapies envisage taking erection sustaining pharmaceutical products orally, among the best known and used being the sidenafil (Viagra^{®}) and the yohimbina (Yohimbine^{®}, Yocon^{®} and others).

Other therapies still envisage taking erection inducing pharmaceutical products by self-injection, by means of the use of a thin and short needle suitable for injecting the pharmaceutical product directly into one of the cavernous bodies of the penis, underneath the glans.

Finally, the most invasive therapies envisage the grafting by surgical operation of suitable prostheses or a reconstructive vascular surgical operation.

All these therapies have a number of drawbacks however.

The identification and the use of a specific therapy is not always in fact easy considering that, often, the erectile disorder is the result of several different problems which are therefore difficult to solve by means of the application of a single therapy.

It must also be emphasised that most of the therapies do not allow obtaining a definitive result and that over time they nearly always create states of discomfort and anxiety which further complicate the emotional conditions of the person.

Nor should it be forgotten as well that treatments such as oral pharmacological therapy or injection pharmacological therapy cause side effects, some of which can be serious.

Surgical operations on the other hand are often excessively traumatic and, besides being invasive, often leave permanent psychological and physical traumas in the person.

Herbal remedies against erectile dysfunction are disclosed in the following references:

Tharakan Binu et al: Phytotherapy Research, vol. 19, no. 6, (2005), pages 457-463, a review of botanical therapies against sexual dysfunction; there is no mention of the herbs of the present application;

Aglarova A M et al: Pharmaceutical Chemistry Journal, vol. 42, no. 2, (2008), pages 81-86, disclosing several medicinal uses of tarragon; use in Tibetan medicine against impotence is mentioned;

WO 89/06966 A, disclosing a herbal tea comprising lemon balm, hawthorn and tanacetum against impotence;

US 2004/1 80104 A1, disclosing a herbal composition against depression; sexual dysfunction is mentioned as a symptom and fennel is listed among many other plants.

### Object of the Invention

The main aim of the present invention is to provide a preparation for the treatment of the erectile disorder that allows overcoming the drawbacks and the contraindications relating to use normally found in traditional treatments. Another object of the present invention is to provide a preparation for the treatment of the erectile disorder that can be used by persons of all ages without any restriction in terms of use.

Another object of the present invention is to provide a preparation for the treatment of the erectile disorder that can be used quickly and in a practical way by the persons involved.

Another object of the present invention is to provide a preparation for the treatment of the erectile disorder which allows maintaining and upgrading the normal physiological erection process up to a late age.

Another object of the present invention is to provide a preparation for the treatment of the erectile disorder which allows to overcome the mentioned drawbacks of the background art in the ambit of a simple, rational, easy, effective to use and low cost solution.

The above objects are achieved by the present preparation for the treatment of the erectile disorder characterised in that it comprises tarragon, lemon balm and fennel mixed with at least one solvent means.

### Embodiments of the Invention

Other characteristics and advantages of the present invention will become more evident from the description of a preferred, but not sole, embodiment of a preparation for the treatment of the erectile disorder.

In the present description, reference is made to a preparation for the treatment of the erectile disorder, usable in particular by a person suffering from such disorder to achieve a normal physiological penis erection process, and to maintain such physiological process for a time interval long enough to have satisfactory sexual intercourse.

The preparation comprises tarragon (according to the binomial nomenclature artemisia dracunculus), lemon balm (melissa officinalis) and fennel (foeniculum vulgare) mixed together using a solvent means in order to obtain a cream to be applied to the person's body.

Usefully, the solvent used is an organic solvent of the glycerol type or the like, commonly used to make creams for cosmetic or pharmaceutical use, if necessary mixed with water.

In particular, the preparation comprises leaves obtained from the lemon balm and tarragon plants, suitably dried and crushed.

From the fennel plant on the other hand the fruits are used, suitably dried and crushed.

The above tarragon, lemon balm, fennel and glycerol are present in the following weight concentrations, assessed with respect to the total weight:
- tarragon between 20% and 40%;
- lemon balm between 20% and 40%;
- fennel between 20% and 40%;
- glycerol between 5% and 20%.

Usefully, by mixing the above compounds, the preparation appears in the form of cream and is ready to use.

Preferably, tarragon, lemon balm, fennel and glycerol are present in the following weight concentrations, assessed with respect to the total weight:
- tarragon 30%;
- lemon balm 30%;
- fennel 30%;
- glycerol 10%.

In particular, for the sole purpose of illustrating the present invention and not in a limitative sense, each 100g of preparation can comprise tarragon, lemon balm, fennel and glycerol in the following quantities:
- tarragon 30g;
- lemon balm 30g;
- fennel 30g;
- glycerol 10g.

The obtained preparation can be suitably mixed with water to obtain the required viscosity.

The preparation thus obtained, in cream form, can preferably be applied on the plantar arch of the person's foot. In fact, on the feet generally and in such plantar arch area in particular numerous nerve endings are present which, when suitably stimulated, produce a reflex action on the entire body.

The preparation, particularly if applied regularly, acts selectively and at the same time on the three systems involved in the physiological erection process: the Central Nervous System (CNS), the axis involving the hypothalamus, the pituitary and gonads and the vascular system.

By acting simultaneously on each of the three systems in fact, the preparation works in order to maintain the correct internal stability condition of the body (homeostasis) indispensable for the normal erective process.

The different properties of tarragon, lemon balm and fennel work in synergy to stimulate the nerve endings at the plantar arch and, by consequently acting on the hypothalamus and on the pituitary, they stimulate the veins and the muscles of the cavernous tissue of the penis responsible for the erection.

In particular, the substances present in the lemon balm (melissa officinalis) leaves act as re-balancers of the nervous system and, specifically, regulate the secretion of the hormones by the pituitary thereby permitting a correct endocrine and metabolic activity of the body.

The fruits of the fennel plant (foeniculum vulgare) possess, on the other hand, substances similar to amphetamine which have a very strong stimulating action on the hypothalamus and on the nerve nuclei, which control the operation of the vascular system which regulates the flow of blood to the cavernous bodies of the penis.

The hypothalamus, in fact, is the brain region involved in regulating the responses of the different organs that take part in maintaining the homeostasis of the body, which is the internal balance condition of the body that ensures a normal biological activity of the tissue cells.

The hypothalamus acts by integrating the signals coming from the outside environment, from other brain areas and from the peripheral organs and consequently regulates the appropriate neuroendocrine responses.

The tarragon (artemisia dracunculus) leaves, thanks to the active ingredients they contain, produce a cleaning, disinfecting and softening action on the area of application, and specifically on the plantar arch, thereby facilitating the absorption of the preparation.

It has in fact been ascertained how the described invention achieves the proposed objects, and in particular the fact is underlined that the use of the preparation does not have the drawbacks and the contraindications normally caused by traditional treatments and can be used by persons of all ages without any restriction in terms of use.

Usefully, the preparation can be used in a quick and practical way by the persons involved.

Furthermore, the preparation allows maintaining and upgrading a normal physical activity until a late age.

## Claims

1. Preparation for use in the treatment of the erectile disorder **characterised by** the fact that it comprises tarragon, lemon balm and fennel mixed with at least one solvent means.

2. Preparation for the use according to claim 1, **characterised by** the fact that said tarragon is in the form of leaves.

3. Preparation for the use according to claim 2, **characterised by** the fact that said tarragon leaves are dried and crushed.

4. Preparation for the use according to claim 1, **characterised by** the fact that said tarragon is present in the weight concentration between 20% and 40%, assessed with respect to the total weight.

5. Preparation for the use according to claim 1, **characterised by** the fact that said lemon balm is in the form of leaves.

6. Preparation for the use according to claim 5, **characterised by** the fact that said lemon balm leaves are dried and crushed.

7. Preparation for the use according to claim 1, **characterised by** the fact that said lemon balm is present in the weight concentration between 20% and 40%, assessed with respect to the total weight.

8. Preparation for the use according to claim 1, **characterised by** the fact that said fennel is in the form of fruits.

9. Preparation for the use according to claim 8, **characterised by** the fact that said fennel fruits are dried and crushed.

10. Preparation for the use according to claim 1, **characterised by** the fact that said fennel is present in the weight concentration between 20% and 40%, assessed with respect to the total weight.

11. Preparation for the use according to claim 1, **characterised by** the fact that said solvent means is of the organic solvent type.

12. Preparation for the use according to claim 1, **characterised by** the fact that said solvent means comprises glycerol.

13. Preparation for the use according to claim 1, **characterised by** the fact that said solvent means is present in the weight concentration between 5% and 20%, assessed with respect to the total weight.

14. Preparation for the use according to claim 12, **characterised by** the fact that said tarragon, said lemon balm, said fennel and said glycerol are present in the following quantities:
| | |
|---|---|
| tarragon | 30g |
| lemon balm | 30g |
| fennel | 30g |
| glycerol | 10g. |

## Patentansprüche

1. Zubereitung zur Verwendung bei der Behandlung der erektilen Dysfunktion, **gekennzeichnet durch** die Tatsache, dass sie Estragon, Zitronenmelisse und Fenchel vermischt mit mindestens einem Lösungsmittel enthält.

2. Zubereitung für die Verwendung nach Anspruch 1, **gekennzeichnet durch** die Tatsache, dass der Estragon in Form von Blättern vorliegt.

3. Zubereitung für die Verwendung nach Anspruch 2, **gekennzeichnet durch** die Tatsache, dass die Estragonblätter getrocknet und zerkleinert sind.

4. Zubereitung für die Verwendung nach Anspruch 1, **gekennzeichnet durch** die Tatsache, dass der Estragon in der Gewichtskonzentration zwischen 20% und 40% bemessen in Bezug auf das Gesamtgewicht vorliegt.

5. Zubereitung für die Verwendung nach Anspruch 1, **gekennzeichnet durch** die Tatsache, dass die Zitronenmelisse in Form von Blättern vorliegt.

6. Zubereitung für die Verwendung nach Anspruch 5, **gekennzeichnet durch** die Tatsache, dass die Zitronenmelisseblätter getrocknet und zerkleinert sind.

7. Zubereitung für die Verwendung nach Anspruch 1, **gekennzeichnet durch** die Tatsache, dass die Zitronenmelisse in der Gewichtskonzentration zwischen 20% und 40% bemessen in Bezug auf das Gesamtgewicht vorliegt.

8. Zubereitung für die Verwendung nach Anspruch 1, **gekennzeichnet durch** die Tatsache, dass der Fenchel in Form von Früchten vorliegt.

9. Zubereitung für die Verwendung nach Anspruch 8, **gekennzeichnet durch** die Tatsache, dass die Früchte getrocknet und zerkleinert sind.

10. Zubereitung für die Verwendung nach Anspruch 1, **gekennzeichnet durch** die Tatsache, dass der Fenchel in der Gewichtskonzentration zwischen 20% und 40% bemessen in Bezug auf das Gesamtgewicht vorliegt.

11. Zubereitung für die Verwendung nach Anspruch 1, **gekennzeichnet durch** die Tatsache, dass das Lösungsmittel von dem organischen Lösungsmitteltyp ist.

12. Zubereitung für die Verwendung nach Anspruch 1, **gekennzeichnet durch** die Tatsache, dass das Lösungsmittel Glycerin umfasst.

13. Zubereitung für die Verwendung nach Anspruch 1, **gekennzeichnet durch** die Tatsache, dass das Lösungsmittel in der Gewichtskonzentration zwischen 5% und 20% bemessen in Bezug auf das Gesamtgewicht vorliegt.

14. Zubereitung für die Verwendung nach Anspruch 12, **gekennzeichnet durch** die Tatsache, dass der Estragon, die Zitronenmelisse, der Fenchel und das Glycerin in den folgenden Mengen vorliegen:
| | |
|---|---|
| Estragon | 30g |
| Zitronenmelisse | 30g |
| Fenchel | 30g |
| Glycerin | 10g. |

## Revendications

1. Préparation destinée à une utilisation dans le traitement des troubles de l'érection, **caractérisée par le fait qu'**elle comprend de l'estragon, de la mélisse et du fenouil mélangés avec au moins un type de moyens de solvant.

2. Préparation destinée à l'utilisation selon la revendication 1, **caractérisé par le fait que** ledit estragon est sous la forme de feuilles.

3. Préparation destinée à l'utilisation selon la revendication 2, **caractérisé par le fait que** lesdites feuilles d'estragon sont séchées et broyées.

4. Préparation destinée à l'utilisation selon la revendication 1, **caractérisé par le fait que** ledit estragon est présent en une concentration en poids comprise entre 20 % et 40 %, évaluée par rapport au poids total.

5. Préparation destinée à l'utilisation selon la revendication 1, **caractérisé par le fait que** ladite mélisse est sous la forme de feuilles.

6. Préparation destinée à l'utilisation selon la revendication 5, **caractérisé par le fait que** lesdites feuilles de mélisse sont séchées et broyées.

7. Préparation destinée à l'utilisation selon la revendication 1, **caractérisé par le fait que** ladite mélisse est présente en une concentration en poids comprise entre 20 % et 40 %, évaluée par rapport au poids total.

8. Préparation destinée à l'utilisation selon la revendication 1, **caractérisé par le fait que** ledit fenouil est sous la forme de fruits.

9. Préparation destinée à l'utilisation selon la revendication 8, **caractérisé par le fait que** lesdits fruits de fenouil sont séchés et broyés.

10. Préparation destinée à l'utilisation selon la revendication 1, **caractérisé par le fait que** ledit fenouil est présent en une concentration en poids comprise entre 20 % et 40 %, évaluée par rapport au poids total.

11. Préparation destinée à l'utilisation selon la revendication 1, **caractérisé par le fait que** lesdits moyens de solvant sont du type solvant organique.

12. Préparation destinée à l'utilisation selon la revendication 1, **caractérisé par le fait que** lesdits moyens de solvant comprennent du glycérol.

13. Préparation destinée à l'utilisation selon la revendication 1, **caractérisé par le fait que** lesdits moyens de solvant sont présents en une concentration en poids comprise entre 5 % et 20 %, évaluée par rapport au poids total.

14. Préparation destinée à l'utilisation selon la revendication 12, **caractérisé par le fait que** ledit estragon, ladite mélisse, ledit fenouil et ledit glycérol sont présents dans les quantités suivantes :
| | |
|---|---|
| estragon | 30g |
| mélisse | 30g |
| fenouil | 30g |
| glycérol | 10g. |
